# EUROPEAN PATENT SPECIFICATION

(11) **EP 2 505 142 B1**
(45) Date of publication and mention of the grant of the patent: **20.07.2016**
(21) Application number: 12161768.2
(22) Date of filing: 28.03.2012
(51) Int. Cl.: A61B 17/04

(54) **Anchor member for looped sutures**
Ankerelement für Schlaufennähte
Élément d'ancrage de sutures en boucle

(30) Priority: 29.03.2011 US 201161468657 P; 14.03.2012 US 201213419476
(43) Date of publication of application: 03.10.2012
(73) Proprietor: Covidien LP, Mansfield, MA 02048 (US)
(72) Inventor: Gleiman, Seth, Branford, CT 06405 (US)
(74) Representative: Soames, Candida Jane

(56) References cited:
- EP-A1- 2 174 598
- EP-A2- 2 149 339
- WO-A1-2005/122954
- WO-A1-2009/009617
- WO-A2-2010/080014
- WO-A2-2012/064902
- US-A- 4 235 238
- US-A1- 2007 049 970
- US-A1- 2008 234 572
- US-A1- 2010 211 098
- US-B1- 6 231 561

## Description

### CROSS-REFERENCE TO RELATED APPLICATION

The present application claims the benefit of and priority to U.S. Provisional Application Serial No. 61/468,657, filed March 29, 2011.

### BACKGROUND

### Technical Field

The present disclosure relates to an anchor assembly comprising an anchor member and a suture having a loop.

### Background of Related Art

Sutures including loops formed therein are known. A sutured loop is typically used to secure the suture to tissue. Traditionally, the loop is formed in a first end of a suture and is configured to receive a second end of the suture. Alternatively, the loop may itself act as an anchor to secure the suture within tissue. Depending on the tissue type, size of the loop and/or the characteristics of the material forming the suture, the loop may not be large and/or stable enough to prevent the suture from pulling through the tissue being sutured. Otherwise, the suture loop may be too large and/or overly rigid to permit placement of the suture in a remote or difficult to access location.

Therefore it would be beneficial to have one or more anchor members capable of reception through the loop in the suture to more readily secure the suture within tissue.

EP 2174598 discloses a suture wound into an end effector about a rod which is then removed before the suture is used in a patient.

EP 2149339 discloses a suture arrangement in which respective ends of the suture engage respective anchors at a point intermediate opposite ends. The anchors include grooves which are used to engage an engagement projection in a needle delivery system to prevent accidental discharge of the anchor.

WO2005/122954 discloses anchor devices including barbs to prevent withdrawal of the anchors after insertion of the anchor into the tissue.

WO2009/009617 discloses a device for the delivery of surgical anchors into a patient.

US6231561 is considered the prior art closest to the subject matter of claim 1, and discloses an anchor assembly with the features of the preamble of claim 1.

### SUMMARY

According to the invention, there is provided an anchor assembly with the features of claim 1. Preferred features are recited in the dependent claims. Anchor members for use with a suture having a loop are provided. The anchor member includes an elongate body having first and second ends. The elongate body is configured for reception within the loop formed in the suture. The anchor member further includes an engagement portion spaced from the first and second ends configured to secure the body portion relative to the loop.

Also provided is an anchor assembly. The anchor assembly includes a suture having a loop formed in the distal end thereof and an anchor member configured to be received through the loop in the suture.

Additionally, a suture anchor kit is provided. The kit includes at least first and second anchor members. Each of the first and second anchor members may include an elongate body having first and second ends. The elongate body may be configured for reception within the loop formed in the suture. The kit may further include an anchor forming apparatus. In one embodiment, at least one of the first and second anchor members is provided on a spool.

### BRIEF DESCRIPTION OF THE DRAWINGS

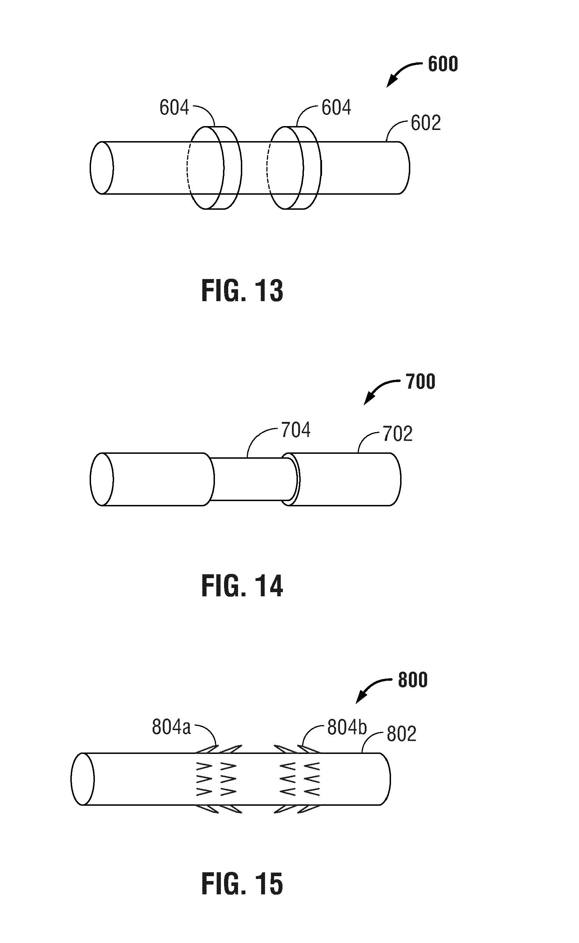

The accompanying drawings, which are incorporated in and constitute a part of this specification, illustrate embodiments of the disclosure and, together with a general description of the disclosure given above, and the detailed description of the embodiments given below, serve to explain the principles of the disclosure. Only figure 15 shows an embodiment of the anchor that forms part of the anchor assembly according to the present invention.
FIG. 1A is a side view of an embodiment of a looped suture for use with one or more anchor members of the present disclosure;
FIG. 1B is a side view of another embodiment of a looped suture for use with one or more anchor members of the present disclosure;
FIG. 2 is a side view of an embodiment of an anchor member according to the present disclosure;
FIG. 3 is a side view of another embodiment of an anchor member according to the present disclosure, including perforations;
FIG. 4 is a side view of still another embodiment of an anchor member according to the present disclosure, including a continuous body;
FIG. 5 is a side view of still another embodiment of an anchor member according to the present disclosure, having a longitudinal core section;
FIG. 6 is a side view of still another embodiment of an anchor member according to the present disclosure, having a plurality of longitudinal core sections;
FIG. 7 is an end view of still yet another embodiment of an anchor member according to the present disclosure, having a plurality of longitudinally extending, radially spaced core sections;
FIG. 8 is a side view of another embodiment of the present disclosure, having rounded ends;
FIG. 9 is a side view of yet another embodiment of the present disclosure, having conical ends;
FIG. 10 is a side view of still another embodiment of the present disclosure, including a rounded first end and a recessed second end;
FIG. 11 is a perspective view of still yet another embodiment of the present disclosure, including a tapered body;
FIGS. 12A-12L are end views of several shaped anchor members according to embodiments of the present disclosure;
FIG. 13 is a side view of another embodiment of an anchor member of the present disclosure;
FIG. 14 is a side view of still another embodiment of an anchor member of the present disclosure;
FIG. 15 is a side view of an anchor member forming part of the anchor assembly of the present invention.
FIG. 16 is a side view of the anchor member of FIG. 2 and the looped suture of FIG. 1B;
FIG. 17 is a partial cross-sectional side view of the anchor member of FIG. 2 received in the opening of the looped suture of FIG. 1B;
FIG. 18 is a perspective view of an anchor forming device of the present disclosure; and
FIG. 19 is a distal end view of the anchor forming device of FIG. 18.

### DETAILED DESCRIPTION

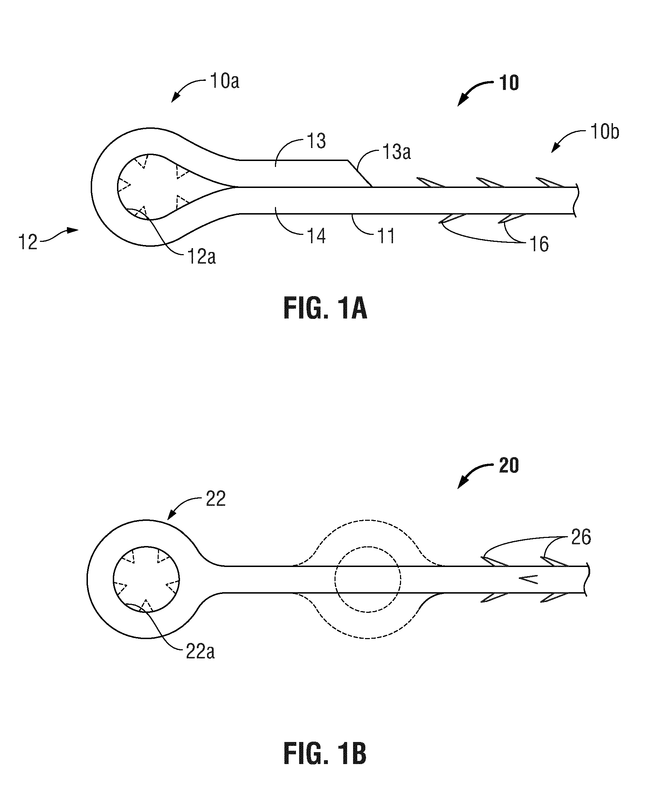

The present disclosure relates to anchors for use with sutures having a loop. With reference initially to FIGS. 1A and 1B, two sutures including a loop are shown generally as sutures 10, 20. Referring initially to FIG. 1A, suture 10 includes a loop 12 formed on a first end 10a of suture 10 by joining together a first section 13 of suture 10 with a second section 14 of suture 10 to define a substantially teardrop opening 12a. First and second sections 13, 14 may be joined using adhesive, welding, bonding or any other suitable method. A proximal end 13a of first section 13 may include a tapered surface 15, as shown, to facilitate reception of suture 10 through an incision. For a more detailed discussion of suture 10, please refer to commonly owned U.S. Patent Application Serial No. 12/548,594, filed August 27, 2009.

Turning briefly to FIG. 1B, in an alternate embodiment, suture 20 includes one or more loops 22 that are molded or otherwise formed along the length of suture 20. Loops 22 define substantially circular openings 22a. Although shown as defining substantially circular openings, it is envisioned that openings 22a may define alternative geometries, including but not limited to, oval, square, rectangular and triangular.

As shown, each of sutures 10, 20 are formed from a monofilament thread 11, 21. It is envisioned, however, that sutures 10, 20 may be formed from braided threads, multifilament threads and other surgical fibers. Although shown having a circular cross-sectional geometry, the cross-sectional geometry of sutures 10, 20 may be of any suitable shape. Either or both of sutures 10, 20 may include one or more needles (not shown) on a second end 10b, 20b thereof. Sutures 10, 20 may also include barbs 16, 26 formed along a length thereof. In one embodiment, sutures 10, 20 may also include barbs (shown in phantom) formed within openings 12a, 22a of loop 12, 22, respectively, to facilitate securement of an anchor member therein. It is envisioned that opening 22a may be tapered to facilitate reception of an anchor member therethrough.

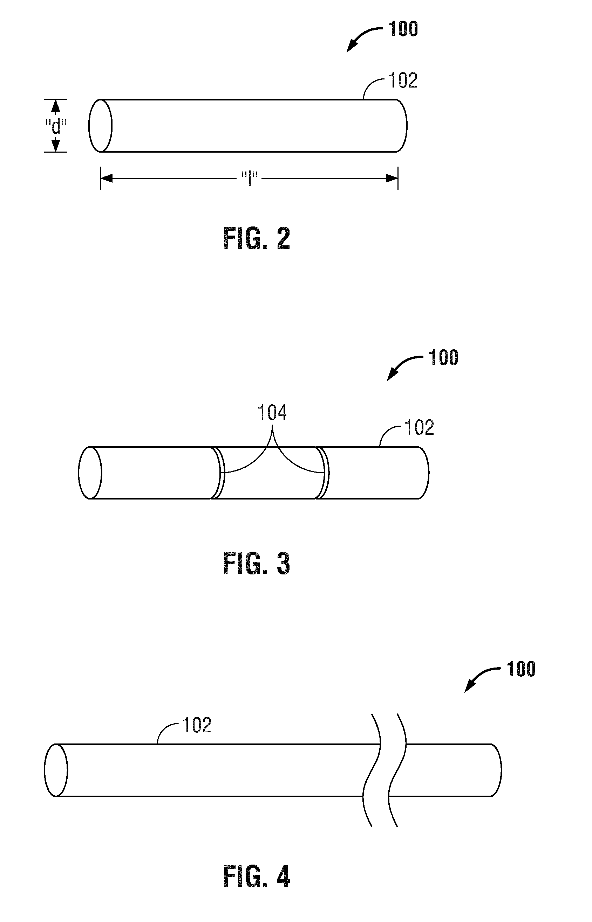

Turning now to FIGS. 3-15, various embodiments of anchor members according to the present disclosure are shown. Although the embodiments of the anchor members will be described herein with reference to either one or both of sutures 10, 20 having loops 12, 22, respectively, it is envisioned that the aspects of the present disclosure may be modified for use with sutures having loops of alternative configurations and/or formed by alternative means.

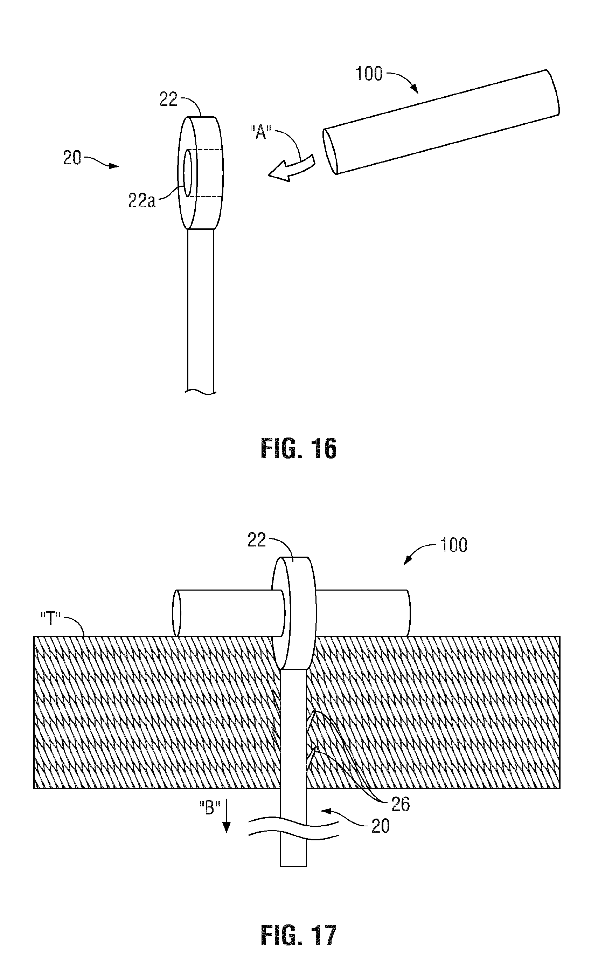

With reference now to FIG. 2, an anchor member according to an embodiment of the present disclosure is shown generally as anchor member 100. Anchor member 100 includes a substantially cylindrical body 102. Cylindrical body 102 includes a diameter "d" configured for reception within loop 12, 22 of suture 10, 20. In one embodiment, cylindrical body 102 is configured for snug reception through loop 12, 22 such that friction maintains anchor 100 within loop 12, 22. Because of the teardrop shape of opening 12a in loop 12, when anchor member 100 is used with suture 10, cylindrical body 102 may include a diameter slightly larger than opening 12a in loop 12. In this manner, loop 12 may be flexed to permit reception of anchor member 100 therethrough. Once anchor member 100 is received within enlarged opening 12a of flexed loop 12, release of the flex force causes loop 12 to return to an unflexed state, thereby contracting loop 12 about cylindrical body 102 of anchor member 100 and securing anchor member 100 within opening 12a of loop 12. Alternatively, cylindrical body 102 may be configured for loose reception through opening 12a, 22a of loop 12, 22, respectively. In this manner, once anchor member 100 has been received through opening 12a, 22a of loop 12, 22, respectively, tension on suture 10, 20 is required to maintain anchor member 100 within opening 12a, 22a of loop 12, 22, respectively. In one embodiment, tension is maintained in suture 10 by engagement of barbs 16, 26 with tissue "T" (FIG. 17). Either or both of cylindrical body 102 or loop 12, 22 of suture 10, 20 may include an adhesive or other coating for securing anchor member 100 within opening 12a, 22a of loop 12, 22, respectively. It is envisioned that the adhesive/coating may be activated by body heat, moisture, exposure to air and/or the application of energy, i.e., light, heat, etc. In yet another embodiment, anchor member 100 and/or loop 12, 22 of suture 10, 22, respectively, may be composed of shape changing material that expands or contracts in response to a given condition. For example, anchor member 100 may be configured to expand when reaching body temperature. In this manner, after anchor member 100 is received through opening 12a, 22a of loop 12, 22, respectively, and is placed in the body of a patient, anchor member 100 expands to secure anchor member 100 with respective openings 12a, 22a of loop 12, 22, respectively. Alternatively, loops 12, 22 may be configured to retract about anchor member 100 when achieving a predetermined condition.

With continued reference to FIG. 2, cylindrical body 102 includes a length "1" sufficient to engage tissue on either side of loop 12, 22 of suture 10, 20, respectively, when anchor member 100 is received through opening 12a, 22a of loop 12, 22, respectively. Anchor member 100 may be provided to a clinician in a predetermined length, as shown in FIG. 2. With reference to FIGS. 3 and 4, in alternative embodiments, anchor member 100 is configured for convenient sizing by a clinician. Referring to FIG. 3, anchor member 100 includes perforations or weakened portions 104 along the length thereof to facilitate shortening of anchor member 100. Perforations may be radially disposed along the cylindrical body 102. Perforations 104 may be configured such that a clinician may break or snap cylindrical body 102 along the perforation 104. Perforations 104 may also, or instead, provide markings upon which a clinician may cut to shorten cylindrical body 102 to a given length. Turning to FIG. 4, anchor member 100 may instead be provided to a clinician on a roll or spool such that anchor member 100 may be unwound and cut to length.

With reference back to FIG. 1, anchor member 100 may be formed of degradable materials, non-degradable materials, and combinations thereof, which may be the same or different from the materials forming suture 10, 20. More particularly, anchor member 100 may be formed of a degradable material selected from the group consisting of polyesters, polyorthoesters, polymer drugs, polydroxybutyrates, proteins, carbonates, homopolymers thereof, copolymers thereof, and combinations thereof. In other embodiments, suitable degradable materials which may be utilized to form anchor member 100 include natural collagenous materials or synthetic resins including those derived from alkylene carbonates such as trimethylene carbonate, tetramethylene carbonate, and the like; caprolactone; dioxanone; glycolic acid; lactic acid; homopolymers thereof; copolymers thereof; and combinations thereof. In some embodiments, glycolide and lactide based polyesters, especially copolymers of glycolide and lactide, may be utilized to form anchor member 100.

Suitable non-degradable materials which may be utilized to form anchor member 100 include polyolefins, such as polyethylene and polypropylene; copolymers of polyethylene and polypropylene, and blends of polyethylene and polypropylene; polyamides (such as nylon); polyamines; polyimines; polyesters such as polyethylene terephthalate; polytetrafluoroethylene; poly ether-esters such as polybutester; polytetramethylene ether glycol; 1,4-butanediol; polyurethanes; and combinations thereof. Other suitable non-degradable materials include silk, cotton, linen, carbon fibers, and the like. The polypropylene may be isotactic polypropylene or a mixture of isotactic and syndiotactic or atactic polypropylene.

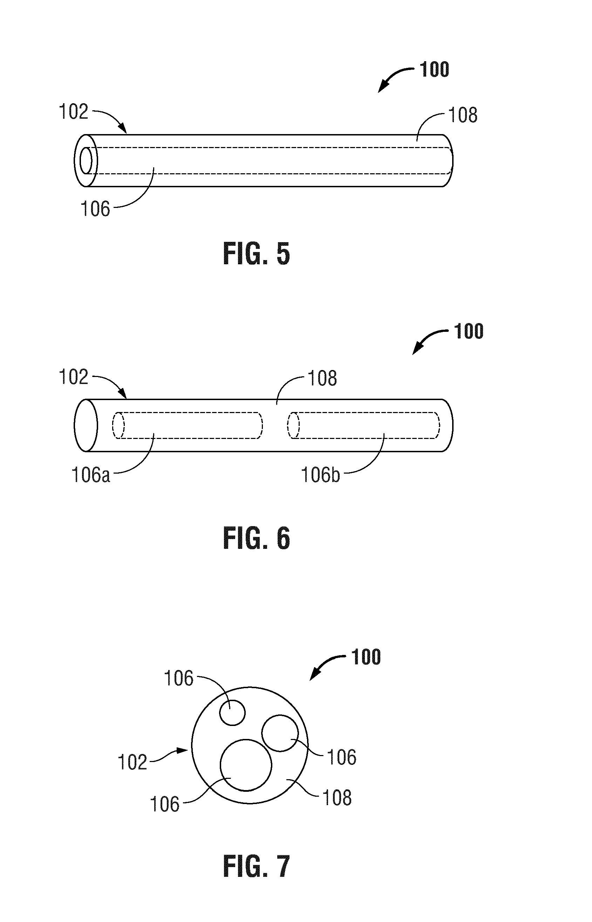

Anchor member 100 may be formed of a single material, or may instead be a composite formed of multiple materials. Turning briefly to FIG. 5, in one embodiment, cylindrical body 102 of anchor member 100 includes a core section 106 and an outer section 108. As shown, core section 106 extends the length of cylindrical body 102. Alternatively, core section 106 may include multiple core section 106a, 106b (FIG. 6). Core sections 106 may each be formed of the same or different materials and those materials may be the same or different than outer section 108. Utilizing composite materials may allow for the anchor member to have desirable mechanical characteristics. It is further envisioned that core section 106 may be left unfilled or empty, thereby creating a hollow anchor member 100. As shown, core sections 106 and outer section 108 are coaxial. With reference to FIG. 7, in an alternative embodiment, cylindrical body 102 of anchor member 100 includes multiple core sections 106 extending along a length of cylindrical body 102. Core sections 106 may extend partially, intermittently or entirely along the length of cylindrical body 120. As shown, core sections 106 are not coaxial and include different diameters.

Anchor member 100 may be formed using any technique within the purview of those skilled in the art, such as, for example, extrusion, molding and/or fiber spinning. In some embodiments, anchor member 100 may include a yarn made of more than one filament, which may contain multiple filaments of the same or different materials. Where anchor member 100 is made of multiple filaments, anchor member 100 may be made using any known technique such as, for example, braiding, weaving or knitting. Anchor member 100 may also be combined to produce a non-woven suture. Anchor member 100 may be drawn, oriented, crinkled, twisted, commingled or air entangled to form yarns as part of the forming process. In one embodiment, a multifilament anchor member may be produced by braiding. The braiding may be done by any method within the purview of those skilled in the art.

Anchor member 100 may include one or more medico-surgically useful substances. In certain embodiments, one or more medico-surgically useful substances may be included in one or more core sections 106 (FIGS. 5-7), as a coating covering cylindrical body 102, impregnated with the material forming cylindrical body 102, and/or otherwise incorporated into anchor member 100. The medico-surgically useful substances may accelerate and/or beneficially modify the healing process and/or otherwise positively affect the outcome of the procedure. In certain embodiments, the substance may be formed from bioactive high molecular weight waxes and oils, natural polymers, proteins, and polysaccharides. The medico-surgically useful substances may be in the form of suspended particulates, dispersible particulates, microspheres, nanospheres, rods, and the like.

Suitable bioactive agents include, for example, biocidal agents, antimicrobial agents, antibiotics, anti-proliferatives, medicants, growth factors, anti-clotting agents, clotting agents, analgesics, anesthetics, anti-inflammatory agents, wound repair agents and the like, chemotherapeutics, biologics, protein therapeutics, monoclonal or polyclonal antibodies, DNA, RNA, peptides, polysaccharides, lectins, lipids, probiotics, diagnostic agents, angiogenics, anti-angiogenic drugs, polymeric drugs, and combinations thereof.

Bioactive agents include substances which are beneficial and tend to promote the healing process. For example, anchor member 100 may be provided with a bioactive agent that will be deposited at the sutured site. The bioactive agent can be chosen for its antimicrobial properties, capability for promoting wound repair and/or tissue growth, or for specific indications such as thrombosis. In some embodiments, combinations of such agents may be applied to anchor member 100 before, during, or after reception within loop 12, 22 of respective sutures 10, 20.

The term "antimicrobial agent" as used herein includes an agent which by itself or through assisting the immune system, helps the body destroy or resist microorganisms which may be pathogenic. An antimicrobial agent includes antibiotics, antiseptics, quorum sensing blockers, antifungals, anti-virals, surfactants, metal ions, antimicrobial proteins and peptides, antimicrobial polysaccharides, disinfectants and combinations thereof. Antimicrobial agents which are slowly released into the tissue can be applied in this manner to aid in combating clinical and sub-clinical infections in a surgical or trauma wound site. In embodiments, suitable antimicrobial agents may be soluble in one or more solvents.

In some embodiments, the following bioactive agents may be used alone or in combination with other bioactive agents described herein: an anthracycline, doxorubicin, mitoxantrone, a fluoropyrimidine, a folic acid antagonist, methotrexate, mitoxantrone, quorum sensing blocker, brominated or halogenated furanones, a podophylotoxin, etoposide, camptothecin, a hydroxyurea, a platinum complex, cisplatin, doxycycline, metronidazole, trimethoprim-sulfamethoxazole, rifamycins like rifampin, a fourth generation penicillin (e.g., a ureidopenicillin a carboxypenicillin, meziocillin, piperacillin, carbenicillin, and ticarcillin, and an analogue or derivative thereof), a first generation cephalosporin (e.g., cephazolin sodium, cephalexin, cefazolin, cephapirin, and cephalothin), a carboxypenicillin (e.g., ticarcillin), a second generation cephalosporin (e.g., cefuroxime, cefotetan, and cefoxitin), a third generation cephalosporin (e.g., naxcel, cefdinir, cefoperazone, ceftazidime, ceftriaxone, and cefotaxime), polyvinyl pyrrolidone (PVP), a fourth generation cephalosporin (e.g., cefepime), a monobactam (e.g., aztreonam), a carbapenem (e.g., imipenem, ertapenem and meropenem), an aminoglycoside (e.g., streptomycin, gentamicin, tobramycin, and amikacin), an MSL group member (e.g., a macrolide, a long acting macrolide, a lincosamide, a streptogramin, erythromycin, azithromycin, clindamycin, syneroid, clarithromycin, and kanamycin sulfate), tetracyclines like minocycline, fusidic acid, trimethoprim, metronidazole; a quinolone (e.g., ciprofloxacin, ofloxacin, gatifloxacin, moxifloxacin, levofloxacin, and trovafloxacin), a DNA synthesis inhibitor (e.g., metronidazole), a sulfonamide (e.g. sulfamethoxazole, trimethoprim, including cefixime, spectinomycin, tetracycline, nitrofurantoin, polymyxin B, and neomycin sulfate), beta-lactam inhibitors like sulbactam, chloramphenicol, glycopeptides like vancomycin, mupirocin, polyenes like amphotericin B, azoles like fluconazole, and other known antimicrobial agent known in the art.

Other suitable bioactive agents include one or more of the following: a fibrosing agent that promotes cell regeneration, a fibrosing agent that promotes angiogenesis, a fibrosing agent that promotes fibroblast migration, a fibrosing agent that promotes fibroblast proliferation, a fibrosing agent that promotes deposition of extracellular matrix, a fibrosing agent that promotes tissue remodeling, a fibrosing agent that is a diverticular wall irritant, silk (such as silkworm silk, spider silk, recombinant silk, raw silk, hydrolyzed silk, acid-treated silk, and acylated silk), talc, chitosan, bleomycin or an analogue or derivative thereof, connective tissue growth factor (CTGF), metallic beryllium or an oxide thereof, copper, saracin, silica, crystalline silicates, quartz dust, talcum powder, ethanol, a component of extracellular matrix, oxidized cellulose, polysaccharides, collagen, fibrin, fibrinogen, poly(ethylene terephthalate), poly(ethylene-co-vinylacetate), N-carboxybutylchitosan, an RGD protein, a polymer of vinyl chloride, cyanoacrylate, crosslinked poly(ethylene glycol)-methylated collagen, an inflammatory cytokine, TGFβ, PDGF, VEGF, TNFa, NGF, GM-CSF, IGF-a, IL-1, IL-8, IL-6, a growth hormone, a bone morphogenic protein, a cell proliferative agent, dexamethasone, isotretinoin, 17-β-estradiol, estradiol, diethylstibesterol, cyclosporine a, all-trans retinoic acid or an analogue or derivative thereof, wool (including animal wool, wood wool, and mineral wool), cotton, bFGF, polyurethane, polytetrafluoroethylene, activin, angiopoietin, insulin-like growth factor (IGF), hepatocyte growth factor (HGF), a colony-stimulating factor (CSF), erythropoietin, an interferon, endothelin-1, angiotensin II, bromocriptine, methylsergide, fibrosin, fibrin, an adhesive glycoprotein, proteoglycan, hyaluronan, secreted protein acidic and rich in cysteine (SPaRC), a thrombospondin, tenacin, a cell adhesion molecule, dextran based particles, an inhibitor of matrix metalloproteinase, magainin, tissue or kidney plasminogen activator, a tissue inhibitor of matrix metalloproteinase, carbon tetrachloride, thioacetamide, superoxide dismutase to scavenge tissue-damaging free radicals, tumor necrosis factor for cancer therapy, colony stimulating factor, interferon, interleukin-2 or other lymphokines to enhance the immune system, platelet rich plasma, thrombin, peptides such as self assembly peptide systems, amino acids such as radA based amino acids, hydrogels such as super absorbing hydrogel materials, combinations thereof, and so forth.

A wide variety of anti-angiogenic factors may be readily utilized within the context of the present disclosure. Representative examples include Anti-Invasive Factor; retinoic acid and derivatives thereof; paclitaxel a highly derivatized diterpenoid; Suramin; Tissue Inhibitor of Metalloproteinase-1; Tissue Inhibitor of Metalloproteinase-2; Plasminogen Activator Inhibitor-1; Plasminogen Activator Inhibitor-2; various forms of the lighter "d group" transition metals such as, for example, vanadium, molybdenum, tungsten, titanium, niobium, and tantalum species and complexes thereof; Platelet Factor 4; Protamine Sulphate (Clupeine); Sulphated Chitin Derivatives (prepared from queen crab shells); Sulphated Polysaccharide Peptidoglycan Complex (SP-PG) (the function of this compound may be enhanced by the presence of steroids such as estrogen, and tamoxifen citrate); Staurosporine; Modulators of Matrix Metabolism, including for example, proline analogs {[(L-azetidine-2-carboxylic acid (LACA), cishydroxyproline, d,L-3,4-dehydroproline, Thiaproline, α,α-dipyridyl, β-aminopropionitrile fumarate; MDL 27032 (4-propyl-5-(4-pyridinyl)-2(3H)-oxazolone; Methotrexate; Mitoxantrone; Heparin; Interferons; 2 Macroglobulin-serum; ChIMP-3; Chymostatin; β-Cyclodextrin Tetradecasulfate; Eponemycin; Camptothecin; Fumagillin Gold Sodium Thiomalate ("GST"); D-Penicillamine ("CDPT"); β-1-anticollagenase-serum; α2-antiplasmin; Bisantrene; Lobenzarit disodium (N-(2)-carboxyphenyl-4-chloroanthronilic acid disodium or "CCA"; Thalidomide; Angostatic steroid; AGM-1470; carboxynaminolmidazole; metalloproteinase inhibitors such as BB94, analogues and derivatives thereof, and combinations thereof.

A wide variety of polymeric drugs may be readily utilized within the context of the present disclosure. Representative examples include steroidal anti-inflammatory agents, non-steroidal anti-inflammatory agents, and combinations thereof. Examples of the non-steroidal anti-inflammatory agent which may be used with the present disclosure are aspirin, indomethacin, ibuprofen, phenylbutazone, diflusinal, and combinations thereof. Examples of the steroidal anti-inflammatory agent which may be used are glucocorticoids such as cortisone and hydrocortisone, betamethasone, dexamethasone, fluprednisolone, prednisone, methylprednisolone, prednisolone, triamcinolone, paramethasone, and combinations thereof.

Although the above bioactive agents have been provided for the purposes of illustration, it should be understood that the present disclosure is not so limited. In particular, although certain bioactive agents are specifically referred to above, the present disclosure should be understood to include analogues, derivatives and conjugates of such agents.

Anchor member 100 may also include, for example, biologically acceptable plasticizers, antioxidants and colorants, which may be impregnated into the filament(s) utilized to form anchor member 100 or included in a coating thereon. Bioactive agents may be applied onto anchor member 100 utilizing any method within the purview of one skilled in the art including, for example, dipping, spraying, vapor deposition, brushing, solvent evaporation, compounding and the like.

Cylindrical body 102 may be dyed in order to increase the visibility of anchor member 100. Any dye suitable for incorporation in medical devices may be used. Such dyes include, but are not limited to, carbon black, bone black, D&C Green No. 6, and D&C Violet No. 2. Filaments in accordance with the present disclosure may be dyed by adding dye in an amount up to about a few percent; in other embodiments, they may be dyed by adding dye in an amount of about 0.2%; in still further embodiments, the dye may be added in an amount from about 0.06% to about 0.08%.

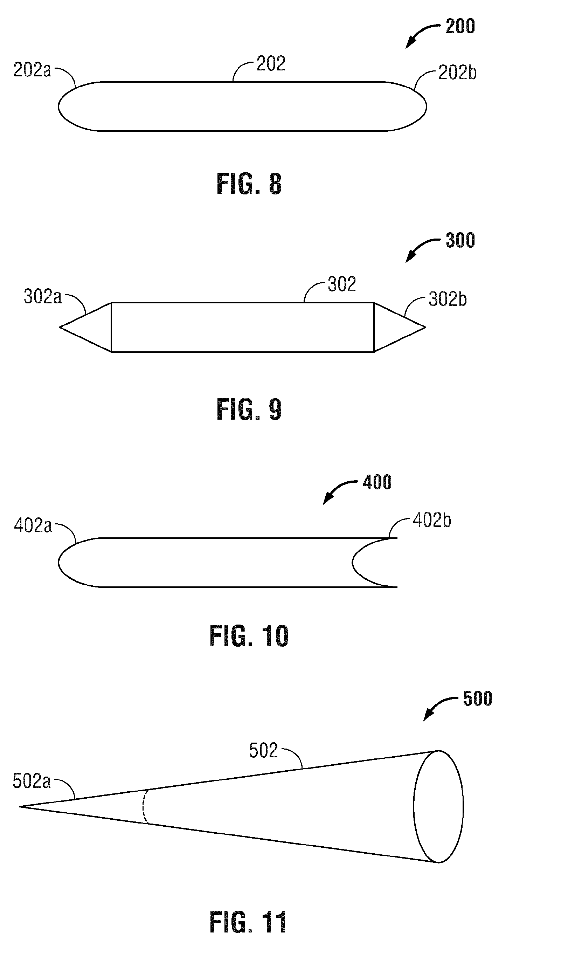

With reference now to FIGS. 8-11, alternate embodiments of anchor members of the present disclosure are shown. With reference initially to FIG. 8, anchor member 200 defines a substantially cylindrical body 202 having rounded first and second ends 202a, 202b. Rounded ends 202a, 202b facilitate reception of anchor member 200 within opening 12a, 22a formed in loop 12, 22, respectively, of respective suture 10, 20.

Turning to FIG. 9, anchor member 300 defines a substantially cylindrical body 302 having conical first and second ends 302a, 302b. As with rounded ends 202a, 202b of anchor member 200, conical first and second ends 302a, 302b of anchor member 300 facilitate reception of anchor member 300 within opening 12a, 22a formed in loops 12, 22, respectively.

With reference to FIG. 10, anchor member 400 defines a substantially cylindrical body 402 having a rounded first end 402a and a recessed second end 402b. Recessed second end 402b is configured to receive a rounded first end 402a of a second anchor member 400. In this manner, anchor members 400 may be longitudinally stacked within a bore of an applicator (not shown) to facilitate storage and manipulation of anchor member 400. Although shown having a recess with a rounded configuration, recessed end 402b of anchor member 400 may include a recess of any shape corresponding in shape to a first end of a second anchor member such that the recess is configured to receive the first end of the second anchor member. For example, an anchor member may include a conical first end, as seen in anchor member 300. As such, the recessed second end would define a conical recess for receiving the conical first end of a second, similarly formed anchor member.

Turning to FIG. 11, anchor member 500 defines a substantially tapered body 502. Tapered body 502 may include a pointed first end 502a, as shown. Alternatively, tapered body 502 may be frustoconical, i.e., first end 502a includes a flattened portion (shown in phantom), to reduce the likelihood of damaging surrounding tissue. The tapered configuration of anchor member 500 facilitates reception of anchor member 500 through opening 12a, 22a of respective loop 12, 22 of suture 10, 20, respectively.

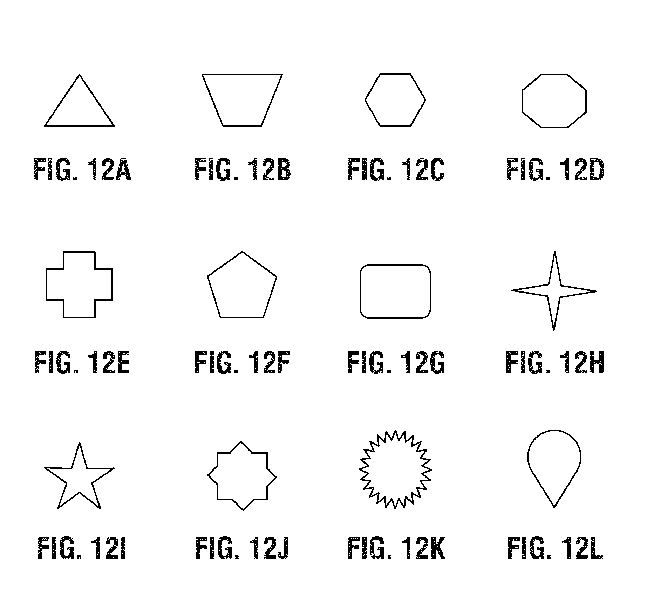

Although shown having a circular cross-sectional profile, embodiments of the present disclosure may include non-circular cross-sections. With reference now to FIGS. 12A-12L, anchor members according to alternative embodiments of the present disclosure are shown. The cross-sectional profile of the anchor members of the present disclosure may be triangular (FIG. 12A), trapezoidal (FIG. 12B), hexagonal (FIG. 12C), octagonal (FIG. 12D), cross-shaped (FIG. 12E), pentagonal (FIG. 12F), rectangular (FIG. 12G), star-shaped (FIGS. 12H-12K), teardrop-shaped (FIG. 12L) or any other suitable cross-sectional configuration. It is further envisioned that any or all of the longitudinal edges of the previously disclosed anchor members may be rounded.

With reference now to FIGS. 13-15, anchor members according to alternative embodiments of the present disclosure are shown. Each of anchor members 600, 700, 800 include an engagement portion configured to engage loop 12, 22 of suture 10, 20, respectively, to secure anchor member 600, 700, 800 within respective openings 12a, 22a of loop 12, 22. In this manner, the engagement portions longitudinally fix or secure the anchor members 600, 700, 800 relative to loop 12, 22. Each of anchor members 600, 700, 800 is substantially similar to anchor member 100 described hereinabove and will only be described as relates to the differences therebetween.

With reference to FIG. 13, anchor member 600 includes a substantially cylindrical body 602 having a pair of raised portions or ridges 604. Ridges 604 are spaced such that loop 12, 22 of suture 10, 20, respectively, may be received therebetween when anchor member 600 is received within respective opening 12a, 22a. Ridges 604 may be integrally formed with cylindrical body 602, or may instead be molded, welded, adhered or otherwise suitably attached thereto. Outer edges of ridges 604 may be rounded and/or tapered to facilitate reception of loop 12, 22 thereover. Inner edges of ridges 604 may be flattened or barbed (not shown) to facilitate securement of loop 12, 22 therebetween. In one embodiment, ridges 604 may be composed of a compressible material to facilitate reception of loop 12, 22 thereover. In this manner, it is further envisioned that ridges 604 may be flattened to permit removal of loop 12, 22 from over the compressed ridge. In this manner, anchor member 600 is selectively secured relative to loop 12, 22 of suture 10, 20, respectively. In alternate embodiments, at least one of the ridges may be removable to enable the anchor member to engage the loop. Once the loop has been engaged, the ridge may then be slid back on the engagement member to lock loop 12, 22 in place.

Turning to FIG. 14, anchor member 700 includes a substantially cylindrical body 702 including a recessed portion or groove 704. Groove 704 is sized to receive loop 12, 22 of suture 10, 20, respectively, when anchor member 700 is received within respective opening 12a, 22a. Groove 704 may be straight or flat, as shown, or may instead be contoured to more securely engaged loop 12, 22, i.e., concave. Anchor member 700 may be formed including groove 704. Alternatively, groove 704 may be cut or otherwise formed in cylindrical body 702 after forming of anchor member 700.

With reference now to FIG. 15, anchor member 800 includes a substantially cylindrical body 802 including first and second sections of spaced barbs 804a, 804b. Barbs 804a extend in a first direction towards an opposite end of cylindrical body 802 and barbs 804b extend in a second, opposite direction towards the other end of cylindrical body 802. Although shown as having a plurality of barbs, it is envisioned that a single barb on either side of loop 12, 22 may be sufficient to retain loop 12, 22 therebetween. Barbs 804a, 804b are configured such that loop 12, 22 may be received over either of barbs 804a, 804b. Once received between barbs 804a, 804b, barbs 804a, 804b are configured to prevent removal of anchor member 800 from within opening 12a, 22a of loop 12, 22, respectively. It is envisioned that either of barbs 804a, 804b may be compressed to permit removal of loop 12, 22 from between barbs 804a, 804b. In this manner, anchor member 800 is selectively secured relative to loop 12, 22 of suture 10, 20, respectively. Barbs 804a, 804b may also be configured to facilitate engagement of anchor member 800 with tissue.

The use of the anchor members according to the present disclosure will now be described with reference to FIGS. 16 and 17. Although reference will be made to anchor member 100 with use of looped suture 20 having a single loop 22, the subtle differences in use between the other anchor members and other looped sutures should become apparent from the following discussion.

As discussed above, anchor member 100 may be provided to a clinician in a predetermined fixed length or may require forming to size by the clinician. Forming anchor member 100 to proper length may require breaking anchor member 100 along a perforation or weakened portion 104 (FIG. 2) or cutting anchor member 100 using shears, clippers or other suitable means. Anchor member 100 may be formed to length prior to or upon receipt through opening 22a of loop 22. It is envisioned that anchor member 100 may be formed to length adjacent tissue "T" (FIG. 17) being sutured.

In one embodiment, and as shown in FIG. 16, once an anchor member of a suitable length is selected and/or formed to size, anchor member 100 is inserted through opening 22a of loop 22, as indicated by arrow "A". Anchor member 100 may be configured to include any of the above described features to facilitate securement within opening 22a of loop 22. Alternatively, and as shown, anchor member 100 is secured within opening 22a of loop 22 upon reception of suture 20 through tissue "T". Tension provided by a clinician, as indicated by arrow "B", engages anchor member 100 with tissue "T". Barbs 26 formed on suture 20 maintain the tension provided by the clinician. As seen in FIG. 17, anchor member 100 prevents suture 20 from being pulled through tissue "T". Once received through opening 22a of loop 22, suture 20 operates as a traditional suture having a traditional anchor or end effector.

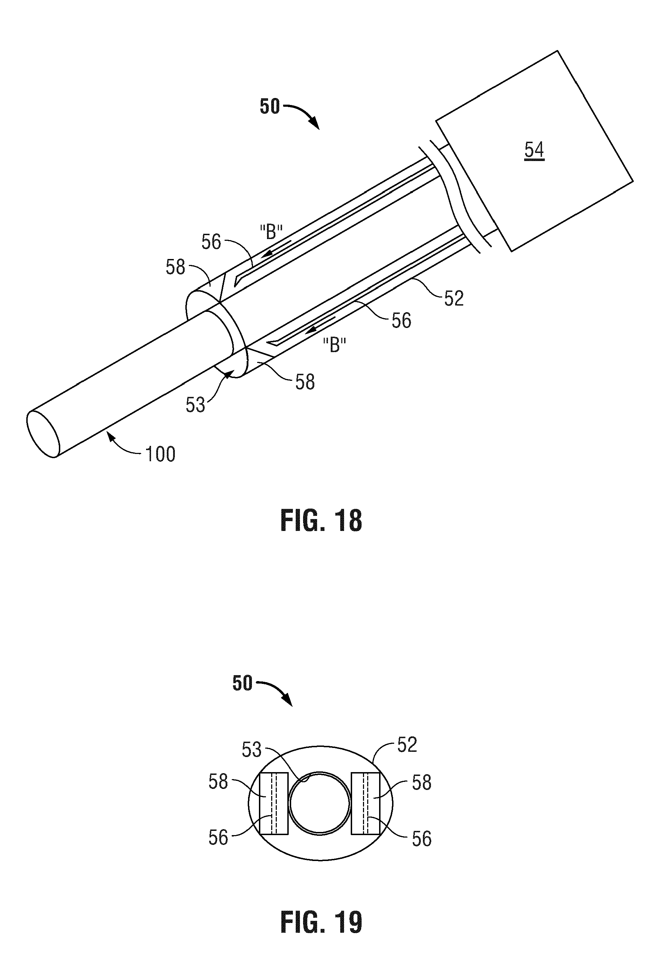

With reference now to FIG. 18 and 19, an apparatus for cutting anchor member 100 to length is shown generally as anchor forming apparatus 50. Forming apparatus 50 includes a substantially cylindrical body 52 defining a bore 53 configured to receive a continuous length of anchor member 100 therethough. Anchor member 100 is provided through bore 53 from an anchor member supply, i.e., roll, (not shown) located in handle assembly 54. Handle assembly 54 is configured to selectively advance anchor member 100 through bore 53. Cutting apparatus 50 further includes a pair of blade members 56. Blade members 56 may include sharpened and/or serrated distal ends. Blade members 56 are configured to be advance, as indicated by arrows "B", to cut anchor member 100 to a desired length. An actuation mechanism (not shown) contained within handle assembly 54 is configured to advance and retract blade members 56. Deflectors 58 located on a distal end 52b of body 52 are configured to deflect blade members 56 towards each other such that anchor member 100 may be severed or cut to a given length. Depending on the angle of deflection, deflectors 58 may be configured such that blades 56 form a straight, rounded, or tapered end on anchor member 100. Handle assembly 54 may further include a heater, ultrasonic generator or other energy producing mechanism operably connected to one or both of blade members 56 for heating and/or oscillating blade members 56 to facilitate cutting of anchor member 100. Cutting apparatus 50 may be configured for use in open or closed procedures and may be modified for use with anchor members of various configurations.

Any one or all of the above described anchor members may be provided in a kit for use by a clinician during a surgical procedure. The kit may include anchor members of predetermined lengths and configurations. The kit may also, or instead, include continuous anchor members provided on roll, spool, or otherwise, for selective sizing by a clinician. The kit may also include scissors, shears or other suitable cutting devices.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope of the disclosure. For example, the previously disclosed anchor members may be composed of first and second independent halves that are configured to be joined within a loop in a suture such that a first end of both halves need not pass through the opening in the loop. In this manner, both first ends may have an enlarged diameter for more securely retaining the anchor member within the opening of the loop.

## Claims

1. An anchor assembly comprising an anchor member (800) and a suture (10,20) having a loop (12, 22),
the anchor member comprising:
an elongate body (802) having first and second ends, and
an engagement portion spaced from the first and second ends configured to secure the elongate body relative to the loop, wherein the engagement portion includes first and second barbed portions (804a, 804b) extending towards each other;
**characterised in that** the elongate body is configured to be received within the loop formed in the suture, the loop being received between the first and second barbed portions to prevent removal of the anchor member (800) from the loop (12, 22).

2. The anchor member of claim 1, wherein the first and second barbed portions each include a single barb.

3. The anchor member of claim 1, wherein the first and second barbed portions each include a plurality of barbs.

4. The anchor member of any preceding claim, wherein the elongate body comprises a bioabsorbable material.

5. The anchor member of any preceding claim, wherein the elongate body is substantially rigid.

6. The anchor member of any preceding claim, wherein the elongate body is substantially cylindrical.

7. The anchor member of any preceding claim, wherein the engagement portion is configured to selectively secure the elongate body relative to the loop.

## Patentansprüche

1. Verankerungsanordnung, umfassend ein Verankerungselement (800) und ein Nahtmaterial (10, 20), das eine Schlaufe (12, 22) aufweist,
wobei das Verankerungselement umfasst:
einen langgestreckten Körper (802), der erste und zweite Enden aufweist, und
einen Eingriffsabschnitt, der von dem ersten und dem zweiten Ende beabstandet ist, konfiguriert, um den langgestreckten Körper relativ zu der Schlaufe zu sichern, wobei der Eingriffsabschnitt erste und zweite mit Widerhaken versehene Abschnitte (804a, 804b) aufweist, die sich aufeinander zu erstrecken,
**dadurch gekennzeichnet, dass** der langgestreckte Körper für die Aufnahme innerhalb der in dem Nahtmaterial ausgebildeten Schlaufe konfiguriert ist, wobei die Schlaufe zwischen den ersten und zweiten mit Widerhaken versehenen Abschnitten aufgenommen wird, um die Entfernung des Verankerungselements (800) aus der Schlaufe (12, 22) zu verhindern.

2. Verankerungselement nach Anspruch 1, wobei die ersten und zweiten mit Widerhaken versehenen Abschnitte jeweils einen einzelnen Widerhaken aufweisen.

3. Verankerungselement nach Anspruch 1, wobei die ersten und zweiten mit Widerhaken versehenen Abschnitte jeweils eine Vielzahl von Widerhaken aufweisen.

4. Verankerungselement nach einem der vorhergehenden Ansprüche, wobei der langgestreckte Körper ein biologisch resorbierbares Material umfasst.

5. Verankerungselement nach einem der vorhergehenden Ansprüche, wobei der langgestreckte Körper im Wesentlichen unelastisch ist.

6. Verankerungselement nach einem der vorhergehenden Ansprüche, wobei der langgestreckte Körper im Wesentlichen zylindrisch ist.

7. Verankerungselement nach einem der vorhergehenden Ansprüche, wobei der Eingriffsabschnitt konfiguriert ist, um den langgestreckten Körper relativ zu der Schlaufe zu sichern.

## Revendications

1. Ensemble d'ancrage comprenant un élément formant ancre (800) et une suture (10, 20) ayant une boucle (12, 22), l'élément formant ancre comprenant :
un corps allongé (802) ayant des première et seconde extrémités, et
une partie de mise en prise espacée des première et seconde extrémités configurée pour fixer le corps allongé par rapport à la boucle, dans laquelle la partie de mise en prise inclut des première et seconde parties barbelées (804a, 804b) s'étendant l'une vers l'autre ;
**caractérisé en ce que** le corps allongé est configuré pour être reçu à l'intérieur de la boucle formée dans la suture, la boucle étant reçue entre les première et seconde parties barbelées pour empêcher l'enlèvement de l'élément formant ancre (800) de la boucle (12, 22).

2. Élément formant ancre selon la revendication 1, dans lequel les première et seconde parties barbelées incluent chacune un picot unique.

3. Élément formant ancre selon la revendication 1, dans lequel les première et seconde parties barbelées incluent chacune une pluralité de picots.

4. Élément formant ancre selon l'une quelconque des revendications précédentes, dans lequel le corps allongé comprend une matière bioabsorbable.

5. Élément formant ancre selon l'une quelconque des revendications précédentes, dans lequel le corps allongé est sensiblement rigide.

6. Élément formant ancre selon l'une quelconque des revendications précédentes, dans lequel le corps allongé est sensiblement cylindrique.

7. Élément formant ancre selon l'une quelconque des revendications précédentes, dans lequel la partie de mise en prise est configurée pour fixer de manière sélective le corps allongé par rapport à la boucle.
